# EUROPÄISCHE PATENTSCHRIFT

(11) **EP 4 114 685 B1**
(45) Veröffentlichungstag und Bekanntmachung des Hinweises auf die Patenterteilung: **31.01.2024**
(21) Anmeldenummer: 22718981.8
(22) Anmeldetag: 30.03.2022
(51) Int. Cl.: B60L 58/12, A61B 5/024, A61B 5/18, A61B 5/00, B60W 40/08, B60W 50/14

(54) **VERFAHREN UND VORRICHTUNG ZUR ERZEUGUNG EINER BENUTZERINDIVIDUALISIERTEN NACHLADEEMPFEHLUNG FÜR EINE BATTERIE EINES ELEKTROFAHRZEUGES**
METHOD AND DEVICE FOR GENERATION OF A PERSONALISED RECHARGING RECOMMENDATION FOR A BATTERY OF ELECTRIC VEHICLES
PROCÉDÉ ET DISPOSITIF POUR GÉNÉRATION D'UNE RECOMMANDATION DE RECHARGE PERSONNALISÉE POUR UNE BATTERIE DES VÉHICULES ÉLECTRIQUES

(30) Priorität: 13.04.2021 DE 102021001931
(43) Veröffentlichungstag der Anmeldung: 11.01.2023
(73) Patentinhaber: Mercedes-Benz Group AG, 70372 Stuttgart (DE)
(72) Erfinder: HANUSCHKIN, Alexander, 71263 Weil der Stadt (DE); HOIS, Joana, 71034 Böblingen (DE); PETERS, Steven, 71701 Schwieberdingen (DE)
(74) Vertreter: Novagraaf Group
(86) Internationale Anmeldenummer: PCT/EP2022/058354
(87) Internationale Veröffentlichungsnummer: WO 2022/218700

(56) Entgegenhaltungen:
- CN-A- 107 521 365
- JP-A- 2020 094 839
- US-A1- 2018 061 415
- US-A1- 2020 239 003

## Beschreibung

Die Erfindung betrifft ein Verfahren zur Erzeugung einer benutzerindividualisierten Nachladeempfehlung für eine Batterie eines Elektrofahrzeuges, bei welchem mindestens ein Zielpunkt bestimmt wird, wobei als Zielpunkt eine Ladestation ausgegeben wird, sowie eine Vorrichtung zur Durchführung des Verfahrens.

Die DE 10 2011 104 153 A1 offenbart ein Verfahren zur Anzeige der Reichweite eines Fahrzeuges mit Elektroantrieb, bei welchem anhand eines aktuellen Ladezustandes der Batterie des Elektroantriebs erreichbare Zielpunkte bestimmt werden und als Zielpunkt mindestens eine Batterieservicestation angegeben wird.

Aus der CN 107 521 365 A ist ein Verfahren zur Wiederaufladung von Elektrofahrzeugen, das auf der Grundlage des wirtschaftlichen Wohlergehens von Arbeitern und Personal des Benutzers optimiert ist.

Die JP 2020 094839 A offenbart eine benutzerindividualisierte Nachladeempfehlung für eine Batterie eines Elektrofahrzeugs, bei der biologische Daten wie Körpertemperatur, Blutdruck etc. eines Nutzers ermittelt und zur Bestimmung eines Zielortes für den Ladevorgang zugrunde gelegt wird.

Aus der US 2018/061415 A1 ist ein Verfahren bekannt, bei dem eine Stimmung eines Nutzers ermittelt wird und an die Stimmung des Nutzers angepasste Rückmeldungen ausgegeben werden. Die Stimmung des Nutzers wird aus der Charakteristik einer Spracheingabe, einem Gesichtsausdruck oder einer Touch-Bedienung ermittelt. Die Rückmeldung umfasst eine Einstellung einer Fahrzeugkomponente oder an eine den Nutzer gerichtete Empfehlung.

Die US 2020/239003 A1 offenbart ein Fahrzeug umfassend eine Vielzahl von Sensoren zur Bestimmung von Parametern in- und außerhalb des Fahrzeugs sowie zur Bestimmung von Fahrzeugnutzer betreffenden Parametern. Ein Prozessmodul verarbeitet Sensordaten wie eine Blickrichtung oder Herzschläge und bestimmt daraus einen durch Emotionen oder Stimmung gekennzeichneten Zustand der Fahrzeugnutzer. Wird eine Verärgerung des Nutzers ermittelt, dann werden Maßnahmen zur Entspannung ergriffen, beispielsweise eine Kaffeepause vorgeschlagen oder eine Ladesäule reserviert.

Aufgabe der Erfindung ist es, ein verbessertes Verfahren zur Erzeugung einer Nachladeempfehlung für eine Batterie eines Elektrofahrzeuges anzugeben, bei welchem die Nachladeempfehlung benutzerindividualisiert ausgegeben wird.

Die Erfindung ergibt sich aus den Merkmalen der unabhängigen Ansprüche. Vorteilhafte Weiterbildungen und Ausgestaltungen sind Gegenstand der abhängigen Ansprüche. Weitere Merkmale, Anwendungsmöglichkeiten und Vorteile der Erfindung ergeben sich aus der nachfolgenden Beschreibung, sowie der Erläuterung von Ausführungsbeispielen der Erfindung, die in den Figuren dargestellt sind.

Die Aufgabe ist mit einem eingangs beschriebenen Verfahren dadurch gelöst, dass eine Empfehlung zur Ansteuerung der Ladestation bei einem von einem Ladezustand der Batterie des Fahrzeuges abhängigen körperlichen und/oder emotionalen Zustand des Fahrers ausgegeben wird. Dies hat den Vorteil, dass die Nachladeempfehlung in Abhängigkeit eines persönlichen Befindens des Fahrers ausgegeben wird, weshalb für verschiedene Fahrer individuelle Nachladevorschläge und/oder-zeitpunkte ausgegeben werden. Da das persönliche Empfinden bei einer vorgegebenen Restreichweite des elektrisch angetriebenen Fahrzeuges von Fahrer zu Fahrer unterschiedlich ist, wird durch die Berücksichtigung des persönlichen Empfindens das Wohlbefinden des Fahrers gesteigert und der Fahrkomfort erhöht. Gegenüber batterieelektrischen Fahrzeugen unsichere Personen werden früher, an elektrisch angetriebene Fahrzeuge gewohnte Personen später informiert, so dass ein Vertrauen gegenüber einer solchen Funktion aufgebaut wird.

Erfindungsgemäß wird der körperliche und/oder emotionale Zustand des Fahrers durch einen beispielsweise aus einer Blickfrequenz auf eine Ladeanzeige, Herzschlag und/oder Blutdruck ermittelten, das Wohlbefinden des Fahrers repräsentierenden Parameter charakterisiert, der in Abhängigkeit zum Ladezustand der Batterie bestimmt wird. Gesichts-, Sprach- oder Physiosignale werden mittels hinreichend bekannter Sensoriken im Fahrzeug ermittelt, so dass auf Zusatzkosten bei einer Installation eines solchen Verfahrens weitgehend verzichtet werden kann.

Der Wert des Ladezustandes der Batterie zur Ausgabe der Empfehlung zur Ansteuerung der Ladestation beim Abfallen des das Wohlbefinden des Fahrers repräsentierenden Parameters oder ab Unterschreitung eines Schwellwertes durch den das Wohlbefinden des Fahrers repräsentierenden Parameter ermittelt. Mit anderen Worten ist der ermittelte Ladezustand dem einen Abfall aufweisenden oder einen Schwellwert unterschreitenden repräsentierenden Parameter des Wohlbefindens zugehörig. Da nun der für das Wohlbefinden kritische Ladezustand bekannt ist, kann der Fahrer rechtzeitig, d.h. mit entsprechendem Vorlauf auf die Ansteuerung einer Ladestation aufmerksam gemacht, bevor das Wohlbefinden in Unwohlsein absinkt.

Erfindungsgemäß wird der Zusammenhang zwischen den das Wohlbefinden des Fahrers ermittelten Werten des mindestens einen Parameters und dem Ladezustand der Batterie mittels einem trainierten Machine Learning Modell ermittelt. Solche Modelle, wie neuronale Netzwerke, Entscheidungsbäume u.ä., sind weitgehend bekannt. Als Eingangsgröße des trainierten Machine Learning Modells dient der aktuellen Ladezustand der Batterie, respektive die Restreichweite des Fahrzeugs und als Ausgangsgröße das aktuell ermittelte Wohlbefinden vorzugsweise dargestellt durch den repräsentierenden Parameter.

In einer Ausgestaltung wird ein Zusammenhang zwischen den anhand des Wohlbefindens des Fahrers ermittelten Werten des Parameters und dem Ladezustand der Batterie mit einer Referenzfunktion, die als Ausgleichs- oder Regressionsfunktion von an verschiedenen Personen ermittelten Werten bestimmt. Somit lässt sich einfach eine Referenzfunktion bestimmen, die als Basisfunktion in jedem Fahrzeug hinterlegt werden kann.

In einer Ausführungsform berücksichtigt das den Zusammenhang von dem das Wohlbefinden des Fahrers repräsentierenden Parameter und dem Ladezustand der Batterie durch Training ermittelte Machine Learning Modell neben dem Ladezustand der Batterie weitere Umgebungsparameter, vorzugsweise Wetter und/oder Außentemperatur und/oder Tages- und/oder Jahreszeit und/oder Verkehrs-/Stausituation. Durch die Verwendung von zwei- oder mehrdimensionalen Modellen mit mehreren Eingangsgrößen wird das Modell verfeinert und das Ergebnis hinsichtlich der Genauigkeit verbessert.

Es ist von Vorteil, wenn die Empfehlung zur Ansteuerung der Ladestation anhand aktueller Umgebungsparameter dynamisch ermittelt wird. Beispielsweise bei einer Wetterverschlechterung und einbrechender Dunkelheit auf einer Route ändert sich der Zusammenhang zwischen Batterieladezustand und dem Wohlbefinden, d.h. dem Fahrzeugnutzer ist zur Vermeidung eines übermäßigen Rückgangs des Wohlbefindens früher eine Empfehlung zur Ansteuerung einer Ladestation auszugeben als bei Tag und gutem Wetter. Daher wird die Nachladeempfehlung immer an die aktuellen Umgebungsbedingungen des Fahrzeuges angepasst und nachgeführt.

In einer weiteren Ausgestaltung wird ein vortrainiertes Modell in jedes Elektrofahrzeug als Startmodell implementiert und vom jeweiligen Fahrer, während eines normalen Fahrbetriebes, personalisiert weitertrainiert. Somit ist das Machine Learning Modell bei der Implementierung in das Fahrzeug bereits vorkonfiguriert, so dass der sich im Fahrzeug anschließende Trainingsprozess schneller ausgeführt werden kann.

In einer weiteren Variante werden Änderungen des Wohlbefindens, hervorgerufen durch kurzzeitige Einflüsse, beim Training des Modells ausgefiltert. Änderungen des Wohlbefindens des Fahrers, die bei Telefonanrufen, Staus u.ä. auftreten, bleiben somit beim personalisierten Training unberücksichtigt.

Ein weiterer Aspekt der Erfindung betrifft eine Vorrichtung zur Erzeugung einer benutzerindividualisierten Nachladeempfehlung für eine Batterie eines Elektrofahrzeuges, umfassend eine Einheit zur Registrierung eines körperlichen oder emotionalen Zustands eines Fahrers und eine Recheneinheit zur Bestimmung einer Restreichweite des Elektrofahrzeuges in Abhängigkeit eines Ladezustandes der Batterie. Bei einer Vorrichtung, mit welcher die Nachladeempfehlung benutzerindividualisiert ausgegeben wird, ist die Einheit zur Registrierung des körperlichen oder emotionalen Zustandes des Fahrers in Abhängigkeit des Batterieladezustandes eingerichtet und eine Recheneinheit zur Ausgabe einer Empfehlung zur Ansteuerung einer Nachladeempfehlung in Abhängigkeit des körperlichen oder emotionalen Zustandes des Fahrers ausgebildet. Erfindungsgemäß ist die Einheit eingerichtet den körperlichen und/oder emotionalen Zustands des Fahrers durch einen aus einer Blickfrequenz auf eine Ladeanzeige, das Wohlbefinden (W) des Fahrers repräsentierenden Parameter zu charakterisieren und in Abhängigkeit zum Ladezustand der Batterie zu bestimmen,
ein trainiertes Machine Learning Modell den Zusammenhang zwischen den das Wohlbefinden (W) des Fahrers ermittelten Werten des mindestens einen Parameters und dem Ladezustand (SOC) der Batterie ermittelt und
die Einheit (5, 7) den Wert des Ladezustandes (SOC) der Batterie zur Ausgabe der Empfehlung zur Ansteuerung der Ladestation beim Abfallen des das Wohlbefinden (W) des Fahrers repräsentierenden Parameters oder ab Unterschreitung eines Schwellwertes durch den das Wohlbefinden (W) des Fahrers repräsentierenden Parameters *aus dem Zusammenhang zwischen Wohlbefinden (W) des Fahrers und dem Ladezustand (SOC)*
*der Batterie* ermittelt, so dass der Fahrer mit entsprechendem Vorlauf auf die Ansteuerung einer Ladestation aufmerksam gemacht wird, bevor das Wohlbefinden (W) in Unwohlsein absinkt.

Weitere Vorteile, Merkmale und Einzelheiten ergeben sich aus der nachfolgenden Beschreibung, in der - gegebenenfalls unter Bezug auf die Zeichnung - zumindest ein Ausführungsbeispiel im Einzelnen beschrieben ist. Beschriebene und/oder bildlich dargestellte Merkmale können für sich oder in beliebiger, sinnvoller Kombination den Gegenstand der Erfindung bilden, gegebenenfalls auch unabhängig von den Ansprüchen, und können insbesondere zusätzlich auch Gegenstand einer oder mehrerer separater Anmeldung/en sein. Gleiche, ähnliche und/oder funktionsgleiche Teile sind mit gleichen Bezugszeichen versehen.

Es zeigen:
- Fig. 1: ein Ausführungsbeispiel der erfindungsgemäßen Vorrichtung in einem elektrisch angetriebenen Fahrzeug,
- Fig. 2: ein erstes Ausführungsbeispiel des erfindungsgemäßen Verfahrens,
- Fig. 3: ein weiteres Ausführungsbeispiel des erfindungsgemäßen Verfahrens,
- Fig. 4: ein weiteres Ausführungsbeispiel des erfindungsgemäßen Verfahrens und
- Fig. 5: ein neuronales Netz im Trainings- und Betriebszustand.

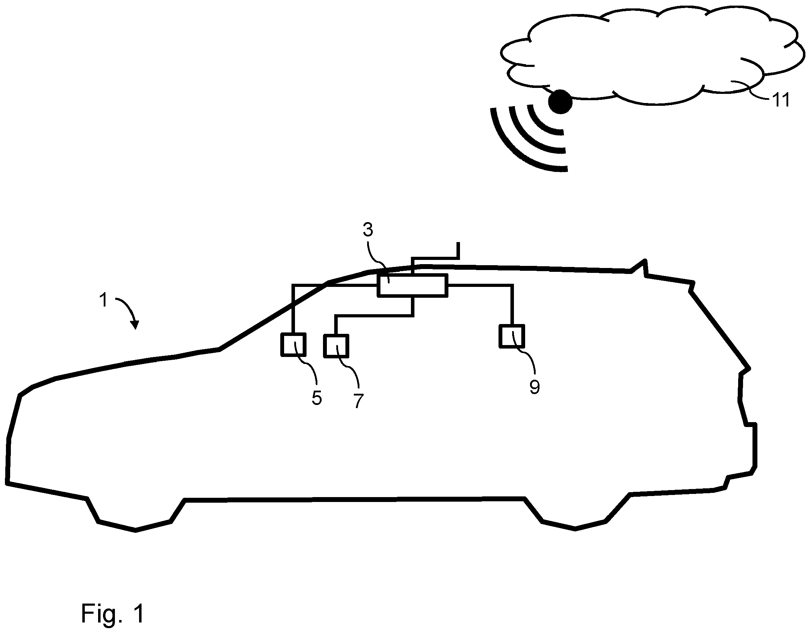

In Fig. 1 ist ein Ausführungsbeispiel der erfindungsgemäßen Vorrichtung in einem elektrisch angetriebenen Fahrzeug dargestellt. Das Fahrzeug 1 umfasst ein Steuergerät 3, welches mit einer Kamera 5 im Innenraum des Fahrzeuges 1 verbunden ist. Die Kamera 5 ist dabei auf das Gesicht des Fahrers gerichtet und für Eye-Tracking, Mood-Detection oder andere Emotisonerkennungssmethoden ausgebildet. Eine den Fahrer ebenfalls überwachende Vitalsensorik 7 und eine nicht dargestellte Fahrstilüberwachungseinrichtung ist an das Steuergerät 3 geführt, genauso wie ein Bordcomputer 9, welcher zur Reichweitenberechnung des elektrisch angetriebenen Fahrzeuges 1 und zur Anzeige des Ladezustandes (State of Charge=SOC) der Batterie ausgebildet ist. Drahtlos kommuniziert das Steuergerät 3 mit einem fahrzeugexternen Backend 11.

Aus den von der Kamera 5 und der Vitalsensorik 7 gelieferten Daten, wie beispielsweise einem Herzschlag, einem Blutdruck sowie einer Blickfrequenz des Fahrers auf eine Ladezustandsanzeige und/oder des Fahrstils ermittelt die Recheneinheit 3 den das Wohlbefinden W des Fahrers charakterisierenden Parameter. Das Wohlbefinden W des Fahrers wird beispielsweise anhand der Blicke des Fahrers auf die Anzeige des Ladezustandes SOC (state of charge) bestimmt. So wird ermittelt, ob sich die individuelle Blickrichtung im Gegensatz zu einem vollen Ladezustand SOC erhöht. Auf Basis der positiven oder negativen Emotionen des Fahrers und der Blickfrequenz auf die Anzeige des Ladezustandes SOC wird das Wohlbefinden W des Fahrers in Abhängigkeit vom SOC und damit der dem SOC zugehörigen Restreichweite mittels einer heuristischen Funktion im Steuergerät 3 ermittelt.

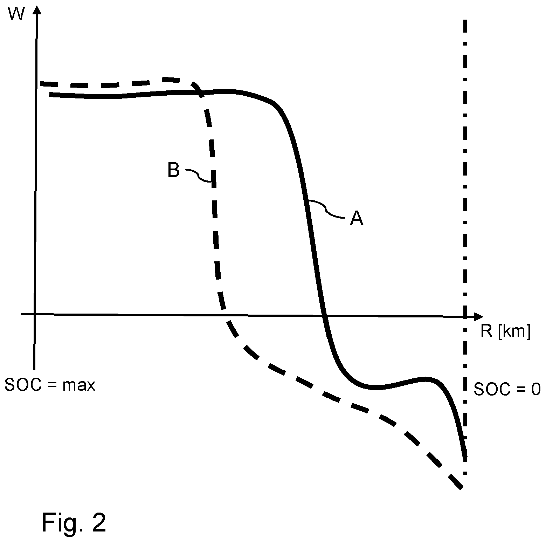

Der von dem Bordcomputer 9 ermittelten SOC bildet eine Eingangsgröße eines Machine Learning Modells, welches in der Recheneinheit 3 abgelegt ist. Das Machine Learning Modell ist zum Beispiel als neuronales Netzwerk ausgebildet, welches erlernt welches Wohlbefinden W des Fahrers einem jeweilige Ladezustand SOC zugehörig ist. Zur funktionellen Approximation des Zusammenhanges wird das Wohlbefinden W als Funktion der verbleibenden Restreichweite R oder des Ladezustandes SOC der Fahrzeugbatterie gesetzt (Fig. 2). Dazu wird ein Zusammenhang zwischen dem das Wohlbefinden W des Fahrers repräsentierenden Parameter und einer Reichweite R des elektrisch angetriebenen Fahrzeuges 1 für verschiedene Fahrer, die durch die Kurven A und B dargestellt sind, trainiert.

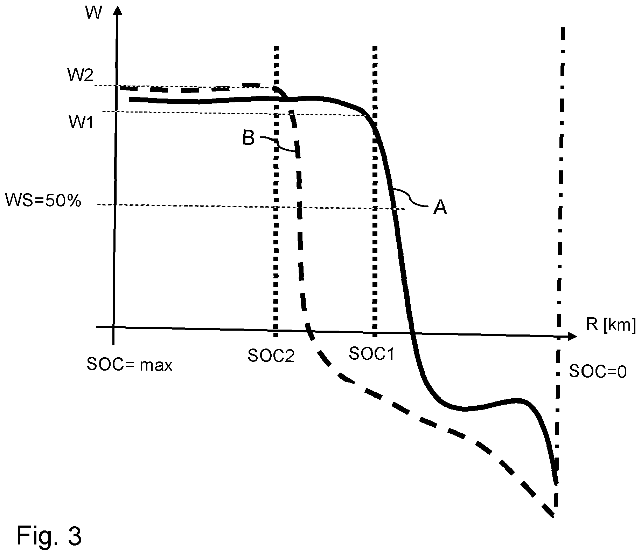

Im Besonderen kann das Machine Learning Modell eine Verringerung des Wohlbefindens eines Fahrers in Abhängigkeit vom SOC vorhersagen. So können lokale Ableitungen der Funktion numerisch berechnet werden und eine starke negative Steigung der Ableitung der Funktion als Indikator oder Schwellwert für ein gefühlsmäßig vom Fahrer empfundenes Abfallen des Wohlbefindens ermittelt und eine rechtzeitige, vor dem Zeitpunkt des Abfalls erfolgende Nachladeempfehlung ausgegeben werden. Alternativ können auch absolute Werte des Wohlbefindens WS des Fahrers als Schwellwert, bspw. bei einer Unterschreitung des Wohlbefindens um einen vorgegebenen Prozentwert von 50%, ausgewertet werden. In Fig. 3 ist hierzu das Wohlbefinden über dem von 100% auf 0 abnehmenden SOC bzw. über der mit Verringerung des Ladezustands einhergehenden abnehmenden Restreichweite R dargestellt. So wird von dem Bordcomputer 9 für die durch die Kurven A, B repräsentierte verschiedenen Fahrer bei einem Abfallen der Steigung des Wohlbefindens Wein Laden beim SOC1 bzw. SOC2 vorgeschlagen, die mit einer nicht dargestellten entsprechenden Restreichweite R2>R1 korrespondieren. Gleichermaßen wäre bei für das Wohlbefinden vorgegebenen, gleich großen absoluten Schwellwerten WS zweier durch die Kurven A, B als W=f(SOC) repräsentierten Fahrern die ermittelte Empfehlung für die nächste Ladestation im Hinblick auf die Restreichweite gemäß den Funktionen unterschiedlich, insbesondere ist in vorliegendem Fall die verbleibende Restreichweite des durch die Kurve B repräsentierten Fahrers größer als des durch die Kurve A repräsentierten Fahrers.

Auf einer geplanten Route werden in Frage kommende Ladestationen im Bereich der bestimmten Restreichweite R1 bzw. R2 auf einer Karte prognostiziert und markiert. D.h. bei einer vorgegebenen Jahreszeit prognostiziert das Machine Learning Modell einen Wohlfühlwert nach XX gefahrenen Kilometern. Dieser bestimmt sich für eine geschätzte Uhrzeit 00.00 ETA (Estimated Time of Arrival) und einem zugehörigen Ladezustand X%. Der Schwellwert für den Wohlfühlwert W1 bzw. W2 gibt dann die entsprechende Nachladeposition vor. Sofern keine Route geplant ist, werden um den Fahrzeugstandort mögliche Ladestationen eingezeichnet, die im Bereich der mit dem entsprechenden Wohlfühlwert W erreichbaren Restreichweite liegen. Den Fahrern wird auf der Fahrt zeitlich vor Abfall des Wohlbefindens W unter W1, W2 (oder alternativ vor Abfall unter den absoluten Schwellwerte WS) ein optischer und akustischer Hinweis auf das erforderliche Ansteuern einer Ladestation bei dem Ladezustand SOC1 und SOC2 und der korrespondierenden Restreichweite gegeben.

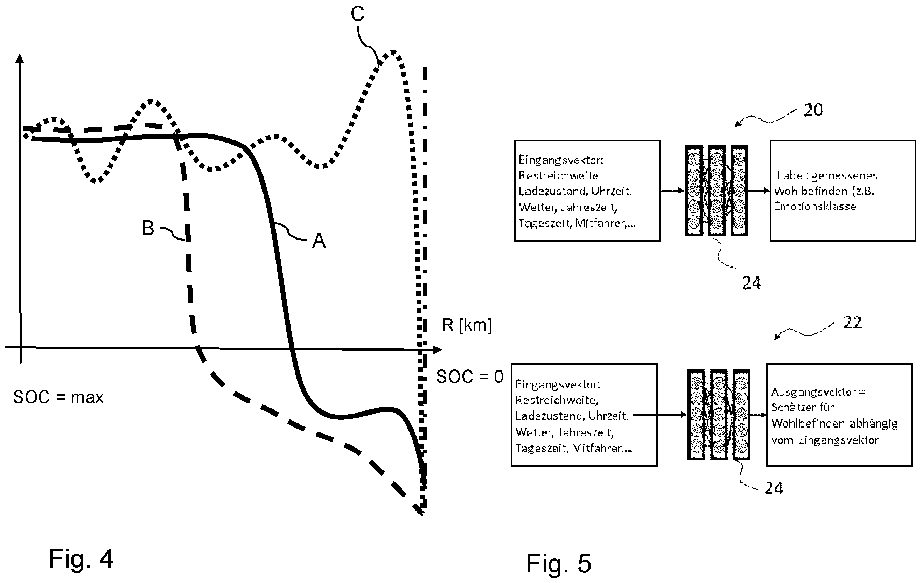

Verfeinert wird das Training des Machine Learning Modells, indem dem Machine Learning Modell Umgebungsbedingungen des Fahrzeuges als Eingangsgrößen zugeführt werden, siehe Fig. 5. Solche Umgebungsbedingungen können das Wetter, die Jahreszeit, die Tageszeit, die Außentemperatur, Anzahl der Mitfahrer oder ähnliches sein, siehe Fig. 5. Im Trainingsmodus 20 werden Gewichte im neuronalen Netz (NN) 24 mit dem Ziel angepasst, die mittlere Abweichung zwischen dem im NN 24 berechnetem Ausgangsvektor und dem Eingangsvektor zugeordneten gemessenen Label über ein gesamtes Trainingsdatenset zu minimieren. Der Eingangsvektor umfasst neben dem SOC die Umgebungsbedingungen, als Label dient das gemessene Wohlbefinden bzw. dessen repräsentativer Wert. Das im Trainingsmodus 20 eintrainierte System gibt den Zusammenhang zwischen dem Eingangsvektor und dem Wohlbefinden wieder. Im Betrieb 22 wird für die eintrainierte Restreichweite und Umgebungsbedingungen aus dem NN 24 das entsprechende Wohlbefinden bestimmt. Alternativ können andere Methoden, z.B. Entscheidungsbäume anstelle des NN 24 angelernt werden.

Beispielsweise liegen als Eingangsgrößen eine Außentemperatur von -10°C und ein Ladezustand SOC der Batterie von 50% vor. Das erlernte Wohlbefinden hat den Wert W1, bei Außentemperaturen von +20°C und einem Ladezustand SOC der Batterie von 50% liegt für das Wohlbefinden ein Wert W2 vor.

Das Machine Learning Modell kann während der Entwicklung des Fahrzeuges vortrainiert werden und als Start-Modell in das Fahrzeug 1 implementiert werden. Das mit dem implementierten Modell erworbene Fahrzeug 1 wird während des normalen Betriebes von Fahrern personalisiert weitertrainiert. Dabei werden Änderungen des Wohlbefindens W, wie Telefonanrufe oder Staus, die kurzfristige Einflüsse auf das Wohlbefinden W ausüben, aus den Eingangsgrößen des Machine Learning Modells herausgefiltert.

Es besteht die Möglichkeit, dass das Machine Learning Modell aus dem Steuergerät 3 verschiedener Fahrzeuge 1 mit dem Backend 11 austauschbar sind. Hierdurch lassen sich vortrainierte Machine Learning Modelle an eine Flotte übermitteln, um die initiale Genauigkeit des Machine Learning Modells zu erhöhen. Durch Klassifizierung vortrainierter Machine Learning Modelle nach Alter, Geschlecht und Erfahrung lassen sich für verschiedene Nutzer personifiziert passende initiale Machine Learning Modelle steuern. Des Weiteren können die individuellen Machine Learning Modelle in dem Backend 11 verglichen werden. So lassen sich Anomalien, wie große Variabilität oder Anstieg des Wohlbefindens W kurz vor Erreichen des Ladezustands 0V der Batterie im emotionalen Verhalten von Fahrern erkennen wie es in Fig. 4 in Kurve C dargestellt ist.

Auf der Fahrt führt das Machine Learning Modell zum Vergleich mit dem Schwellwert das Wohlbefindens W dynamisch nach. So können bei einsetzendem Regen und Dunkelheit entsprechende Modelldaten der Bestimmung des Abfalls des Wohlbefindens zu Grunde gelegt werden. Das Wohlbefinden W wird als Funktion des Wetters, der Außentemperatur, der Tageszeit ETA und der zurückgelegten Fahrstrecke dynamisch nachgeführt, d.h. bei einsetzendem Regen verkürzt sich entsprechend der gelernten Daten die Fahrstrecke, die mit dem Wohlfühlwert oberhalb eines Schwellwertes erreichbar ist.

In einer anderen Ausführung lernt das Machine Learning Modell, welches das Wohlbefinden W als Parameter des Ladezustandes SOC, der Tageszeit und der Jahreszeit approximiert, dass beispielsweise in den frühen Morgenstunden das Wohlbefinden W des Fahrers zwar auf einem niedrigen Niveau liegt, aber unabhängig vom Ladezustand SOC ist, da das Fahrzeug 1 zum Pendeln zur Arbeitsstätte genutzt wird und am Arbeitsplatz geladen werden kann. Das Machine Learning Modell lernt aber auch, dass im Winter nach 9 Uhr das Wohlbefinden des Fahrers sehr stark mit dem Ladezustand SOC der Batterie korreliert, da hier das Fahrzeug 1 beispielsweise zu Lieferdiensten über längere Strecken eingesetzt wird und der Fahrer bereits bei halb beladenem Fahrzeug 1 gestresst sein kann.

Mit der beschriebenen Lösung kann das emotionale Empfinden des Fahrers dahingehend verändert werden, dass Ladezeitpunkte bei einem niedrigen Ladezustand SOC der Batterie vom Fahrer toleriert werden können, da er erfahren hat, dass eine angenehme Fahrt ohne Reichweitenängste möglich ist.

## Patentansprüche

1. Verfahren zur Erzeugung einer benutzerindividualisierten Nachladeempfehlung für eine Batterie eines Elektrofahrzeuges, bei welchem mindestens ein Zielpunkt bestimmt wird, wobei als Zielpunkt eine Ladestation ausgegeben wird,
eine Empfehlung zur Ansteuerung der Ladestation bei einem von einem Ladezustand (SOC) der Batterie des Fahrzeuges (1) abhängigen körperlichen und/oder emotionalen Zustand des Fahrers ausgegeben wird, **dadurch gekennzeichnet, dass**
der körperliche und/oder emotionale Zustand des Fahrers aus den von einer Kamera (7) und einer Vitalsensorik (9) gelieferten Daten anhand einer Blickfrequenz des Fahrers auf eine Ladezustandanzeige das Wohlbefinden (W) des Fahrers repräsentierenden Parameter charakterisiert wird, der in Abhängigkeit zum Ladezustand der Batterie bestimmt wird,
der Zusammenhang zwischen den das Wohlbefinden (W) des Fahrers ermittelten Werten des mindestens einen Parameters und dem Ladezustand (SOC) der Batterie mittels einem trainierten Machine Learning Modell ermittelt wird und der Wert des Ladezustandes (SOC) der Batterie zur Ausgabe der Empfehlung zur Ansteuerung der Ladestation beim Abfallen des das Wohlbefinden (W) des Fahrers repräsentierenden Parameters oder ab Unterschreitung eines Schwellwertes durch den das Wohlbefinden (W) des Fahrers repräsentierenden Parameters *aus dem Zusammenhang zwischen Wohlbefinden (W) des Fahrers und dem Ladezustand (SOC) der Batterie ermittelt wird,*
so dass der Fahrer mit entsprechendem Vorlauf auf die Ansteuerung einer Ladestation aufmerksam gemacht wird, bevor das Wohlbefinden (W) in Unwohlsein absinkt.

2. Verfahren nach Anspruch 1,
**dadurch gekennzeichnet, dass**
ein Zusammenhang zwischen den anhand des Wohlbefindens (W) des Fahrers ermittelten Werten des Parameters und dem Ladezustand (SOC) der Batterie mit einer Referenzfunktion an verschiedenen Personen ermittelten Werten bestimmt wird.

3. Verfahren nach Anspruch 1 oder 2,
**dadurch gekennzeichnet, dass**
das den Zusammenhang von dem das Wohlbefinden (W) des Fahrers repräsentierenden Parameter und dem Ladezustand (SOC) der Batterie durch Training ermittelnde Machine Learning Modell neben dem Ladezustand (SOC) der Batterie weitere Umgebungsparameter, vorzugsweise Wetter und/oder Außentemperatur und/oder Tages- und/oder Jahreszeit und/oder Verkehrssituation berücksichtigt.

4. Verfahren nach einem der vorhergehenden Ansprüche,
**dadurch gekennzeichnet, dass**
die Empfehlung zur Ansteuerung der Ladestation anhand aktueller Umgebungsparameter dynamisch ermittelt wird.

5. Verfahren nach mindestens einem der vorhergehenden Ansprüche,
**dadurch gekennzeichnet, dass**
ein vortrainiertes Modell in jedes Elektrofahrzeug (1) als Startmodell implementiert und vom jeweiligen Fahrer, während eines normalen Fahrbetriebes, personalisiert weitertrainiert wird.

6. Verfahren nach mindestens einem der vorhergehenden Ansprüche,
**dadurch gekennzeichnet, dass**
Änderungen des Wohlbefindens (W), hervorgerufen durch kurzzeitige Einflüsse, beim Training des Modells ausgefiltert werden.

7. Vorrichtung zur Erzeugung einer benutzerindividualisierten Nachladeempfehlung einer Batterie eines Elektrofahrzeuges, umfassend eine Einheit (5, 7) zur Registrierung eines körperlichen oder emotionalen Zustands eines Fahrers und eine Recheneinheit (3) zur Bestimmung einer Restreichweite (R) des Elektrofahrzeuges (1) in Abhängigkeit eines Ladezustandes (SOC) der Batterie, wobei
die Einheit (5, 7) zur Registrierung des körperlichen oder emotionalen Zustandes des Fahrers in Abhängigkeit des Ladezustandes (SOC) der Batterie eingerichtet ist und eine Recheneinheit (3) zur Ausgabe einer Empfehlung zur Ansteuerung einer Nachladeempfehlung in Abhängigkeit des körperlichen oder emotionalen Zustandes des Fahrers ausgebildet ist.
**dadurch gekennzeichnet, dass**
die Recheneinheit (3) eingerichtet ist den körperlichen und/oder emotionalen Zustands des Fahrers aus den von einer Kamera (7) und einer Vitalsensorik (9) gelieferten Daten anhand einer Blickfrequenz auf eine Ladezustandsanzeige
das Wohlbefinden (W) des Fahrers repräsentierenden Parameter zu charakterisieren und in Abhängigkeit zum Ladezustand der Batterie zu bestimmen, ein trainiertes Machine Learning Modell in der Recheneinheit (3) den Zusammenhang zwischen den das Wohlbefinden (W) des Fahrers ermittelten Werten des mindestens einen Parameters und dem Ladezustand (SOC) der Batterie ermittelt und
die Recheneinheit (3) den Wert des Ladezustandes (SOC) der Batterie zur Ausgabe der Empfehlung zur Ansteuerung der Ladestation beim Abfallen des das Wohlbefinden (W) des Fahrers repräsentierenden Parameters oder ab Unterschreitung eines Schwellwertes durch den das Wohlbefinden (W) des Fahrers repräsentierenden Parameters *aus dem Zusammenhang zwischen Wohlbefinden (W) des Fahrers und dem Ladezustand (SOC) der Batterie* ermittelt, so dass der Fahrer mit entsprechendem Vorlauf auf die Ansteuerung einer Ladestation aufmerksam gemacht wird, bevor das Wohlbefinden (W) in Unwohlsein absinkt.

## Claims

1. Method for generating a user-individualized recharging recommendation for a battery of an electric vehicle, in which at least one target point is determined, a charging station being output as the target point,
a recommendation for activating the charging station being output in a physical and/or emotional state of the driver dependent on a state of charge (SOC) of the battery of the vehicle (1), **characterized in that**
the physical and/or emotional state of the driver from the data supplied by a camera (7) and a vital sensor system (9) is characterized on the basis of a parameter representing a viewing frequency of the driver with respect to a state-of-charge display, the well-being (W) of the driver, which parameter is determined depending on the state of charge of the battery,
the relationship between the values, ascertained the well-being (W) of the driver, of the at least one parameter and the state of charge (SOC) of the battery is ascertained by means of a trained machine learning model, and the value of the state of charge (SOC) of the battery for outputting the recommendation for activating the charging station when the parameter representing the well-being (W) of the driver falls or from the point at which a threshold value is not met by the parameter representing the well-being (W) of the driver is *ascertained from the relationship between the well-being (W) of the driver and the state of charge (SOC) of the battery,*
so that the driver is alerted to the activation of a charging station with an appropriate lead time before the well-being (W) drops to discomfort.

2. Method according to claim 1,
**characterized in that**
a relationship between the values of the parameter ascertained on the basis of the well-being (W) of the driver and the state of charge (SOC) of the battery is determined using a reference function at different persons ascertained values.

3. Method according to either claim 1 or claim 2,
**characterized in that**
the machine learning model ascertaining the relationship between the parameter representing the well-being (W) of the driver and the state of charge (SOC) of the battery takes into account, in addition to the state of charge (SOC) of the battery, further environmental parameters, preferably weather and/or outside temperature, and/or time of day and/or season and/or traffic situation.

4. Method according to any of the preceding claims,
**characterized in that**
the recommendation for activating the charging station is dynamically ascertained using current environmental parameters.

5. Method according to at least one of the preceding claims,
**characterized in that**
a pretrained model is implemented in each electric vehicle (1) as a start model and is further trained in a personalized manner by the corresponding driver, during a normal driving operation.

6. Method according to at least one of the preceding claims,
**characterized in that**
changes in the well-being (W) caused by short-term influences are filtered out during training of the model.

7. Device for generating a user-individualized recharging recommendation of a battery of an electric vehicle, comprising a unit (5, 7) for registering a physical or emotional state of a driver and a computing unit (3) for determining a remaining range (R) of the electric vehicle (1) depending on a state of charge (SOC) of the battery,
the unit (5, 7) being designed to register the physical or emotional state of the driver depending on the state of charge (SOC) of the battery, and a computing unit (3) being designed to output a recommendation for activating a recharging recommendation depending on the physical or emotional state of the driver.
**characterized in that**
the computing unit (3) is designed to characterize the physical and/or emotional state of the driver from the data supplied by a camera (7) and a vital sensor system (9) on the basis of a viewing frequency with respect to a state-of-charge display the parameter representing the well-being (W) of the driver, and to determine said parameter depending on the state of charge of the battery, a trained machine learning model in the computing unit (3) ascertains the relationship between the values, ascertained from the well-being (W) of the driver, of the at least one parameter and the state of charge (SOC) of the battery, and
the computing unit (3) ascertains the value of the state of charge (SOC) of the battery for outputting the recommendation for activating the charging station when the parameter representing the well-being (W) of the driver falls or from the point at which a threshold value is not met by the parameter representing the well-being (W) of the driver *from the relationship between the well-being (W) of the driver and the state of charge (SOC) of the battery,* so that the driver is alerted to the activation of a charging station with an appropriate lead time before the well-being (W) drops to discomfort.

## Revendications

1. Procédé permettant la génération d'une recommandation de recharge individualisée par l'utilisateur pour une batterie d'un véhicule électrique, dans lequel au moins une destination est déterminée, dans lequel une station de charge est émise comme destination,
une recommandation pour le guidage vers la station de charge est émise en fonction d'un état physique et/ou émotionnel du conducteur dépendant d'un état de charge (SOC) de la batterie du véhicule (1), **caractérisé en ce que**
l'état physique et/ou émotionnel du conducteur est **caractérisé par** un paramètre représentant le bien-être (W) du conducteur à partir des données fournies par une caméra (7) et un système de capteurs vitaux (9) à l'aide d'une fréquence de regards du conducteur en direction d'un affichage d'état de charge, lequel paramètre est déterminé en fonction de l'état de charge de la batterie,
la relation entre les valeurs de l'au moins un paramètre définies par le bien-être (W) du conducteur et l'état de charge (SOC) de la batterie est définie au moyen d'un modèle d'apprentissage automatique formé et la valeur de l'état de charge (SOC) de la batterie pour l'émission de la recommandation pour le guidage vers la station de charge est définie à partir de la relation entre le bien-être (W) du conducteur et l'état de charge (SOC) de la batterie en cas de chute du paramètre représentant le bien-être (W) du conducteur ou à partir de la non-atteinte d'une valeur seuil par le paramètre représentant le bien-être (W) du conducteur,
de sorte que le conducteur est averti suffisamment à l'avance du guidage vers une station de charge avant que le bien-être (W) ne diminue jusqu'à atteindre l'inconfort.

2. Procédé selon la revendication 1,
**caractérisé en ce que**
une relation est déterminée entre les valeurs du paramètre définies à l'aide du bien-être (W) du conducteur et l'état de charge (SOC) de la batterie avec une fonction de référence de valeurs définies avec différentes personnes.

3. Procédé selon la revendication 1 ou 2,
**caractérisé en ce que**
le modèle d'apprentissage automatique définissant la relation entre le paramètre représentant le bien-être (W) du conducteur et l'état de charge (SOC) de la batterie par formation prend en compte, outre l'état de charge (SOC) de la batterie, d'autres paramètres d'environnement, de préférence la météo et/ou la température extérieure et/ou le moment de la journée et/ou la saison et/ou la situation du trafic.

4. Procédé selon l'une des revendications précédentes,
**caractérisé en ce que**
la recommandation pour le guidage vers la station de charge est définie de manière dynamique à l'aide de paramètres d'environnement actuels.

5. Procédé selon au moins l'une des revendications précédentes, **caractérisé en ce que**
un modèle préentraîné est mis en oeuvre dans chaque véhicule électrique (1) en tant que modèle de départ et est ensuite formé de manière personnalisée par le conducteur respectif pendant un mode de conduite normal.

6. Procédé selon au moins l'une des revendications précédentes, **caractérisé en ce que**
des changements du bien-être (W), causés par des influences à court terme, sont filtrés lors de la formation du modèle.

7. Dispositif permettant la génération d'une recommandation de recharge individualisée par l'utilisateur d'une batterie d'un véhicule électrique, comprenant une unité (5, 7) permettant d'enregistrer un état physique ou émotionnel d'un conducteur et une unité de calcul (3) permettant de déterminer une autonomie restante (R) du véhicule électrique (1) en fonction d'un état de charge (SOC) de la batterie,
dans lequel
l'unité (5, 7) est configurée pour enregistrer l'état physique ou émotionnel du conducteur en fonction de l'état de charge (SOC) de la batterie et une unité de calcul (3) est configurée pour émettre une recommandation pour la commande d'une recommandation de recharge en fonction de l'état physique ou émotionnel du conducteur,
**caractérisé en ce que**
l'unité de calcul (3) est configurée pour caractériser l'état physique et/ou émotionnel du conducteur à partir des données fournies par une caméra (7) et un système de capteurs vitaux (9) à l'aide d'une fréquence de regards en direction d'un affichage d'état de charge, un paramètre représentant le bien-être (W) du conducteur, et pour le déterminer en fonction de l'état de charge de la batterie, un modèle d'apprentissage automatique formé dans l'unité de calcul (3) définit la relation entre les valeurs de l'au moins un paramètre définies par le bien-être (W) du conducteur et l'état de charge (SOC) de la batterie et
l'unité de calcul (3) définit, à partir de la relation entre le bien-être (W) du conducteur et l'état de charge (SOC) de la batterie, la valeur de l'état de charge (SOC) de la batterie pour l'émission de la recommandation pour le guidage vers la station de charge en cas de chute du paramètre représentant le bien-être (W) du conducteur ou à partir de la non-atteinte d'une valeur seuil par le paramètre représentant le bien-être (W) du conducteur, de sorte que le conducteur est averti suffisamment à l'avance du guidage vers une station de charge avant que le bien-être (W) ne diminue jusqu'à atteindre l'inconfort.
